# EUROPEAN PATENT APPLICATION

(11) **EP 1 982 712 A1**
(43) Date of publication of application: **22.10.2008**
(21) Application number: 06757779.1
(22) Date of filing: 06.06.2006
(51) Int. Cl.: A61K 31/4025

(54) **PHARMACEUTICAL FORMULATIONS IN THE FORM OF SOLIDS, SEMISOLIDS, IN SUSPENSION, IN SOLUTION, IN EMULSION OR IN SYRUP, CONTAINING CLINDAMYCIN AND ONE OR MORE MEMBERS OF THE AZOLE FAMILY**

(30) Priority: 12.08.2005 MX PA05008571
(71) Applicant: WORLD-TRADE IMPORT-EXPORT, WTIE, A.G., 6302 Zug (CH)
(72) Inventor: GARCÍA ARMENTA, María Elena, C.P. 45020 Guadalajara, Jalisco, México (MX); GARCÍA ARMENTA, Patricia, C.P. 45110 Zapopan, Jalisco, México (MX); ÁLVAREZ OCHOA, Víctor Guillermo, C.P. 45222 Zapopan, Jalisco, México (MX); SANTOS MURILLO, Josefina, C.P. 44600 Zapopan, Jalisco, México (MX); GARCÍA ALCALA, Martin Omar, C.P. 44410 Guadalajara, Jalisco, México (MX); ÁLVAREZ ÁLVAREZ, Aracely, C.P. 45235 Tlaquepaque, Jalisco, México (MX); ESPINOSA ABDALA, Leopoldo de Jesus, Col. colinas de San Javier C.P. 45110 Zapopan, Jalisco (MX)
(74) Representative: Carvajal y Urquijo, Isabel
(86) International application number: PCT/MX2006/000046
(87) International publication number: WO 2007/021167

(57) **Abstract**

The invention relates to the pharmaceutical industry in general and to the pharmaceutical industry involved in the production of various different pharmaceutical formulations containing clindamycin. The invention essentially consists of solid pharmaceutical formulations containing clindamycin and is **characterized in that** it comprises: (a) one or more members of the azole family, (b) one or more diluent agents, (c) one or more disintegrating agents, (d) one or more binding agents, (e) one or more solvents, (f) one or more lubricants, (g) one or more anti-adherents, (h) one or more solubilising agents, (i) one or more surfactants, (j) one or more emulsifying agents, (k) one or more sweetening agents, (l) one or more flavoring agents and/or essences, (m) one or more antioxidants, (n) one or more antimicrobial agents, and (o) one or more viscosifying agents.

## Description

### FIELD OF THE INVENTION

The present invention is related generally to the pharmaceutical industry, and particularly to the pharmaceutical industry that produces various pharmaceutical formulations containing Clindamycin and one or more members from the azole family.

### BACKGROUND OF THE INVENTION

Solid pharmaceutical forms are preparations that contain the active principle(s) and additives, generally disc-shaped, scored or non-scored, molded and of variable size obtained by compressing powders or granules into tablets, capsules, patches, pessaries, beads, suppositories, troches or lozenges.

A solution is a clear and homogeneous liquid preparation obtained by dissolving the active principle(s) and additives in water, and which is employed for external or internal use.

A semi-solid is a preparation that up to certain extent is a solid having stiffness and a viscosity between that of a solid and a liquid.

A syrup is an aqueous solution with a high content of carbohydrates such as: sucrose, sorbitol, dextrose, etc, of viscous consistency in which the active principle(s) and the additives are dissolved.

Suspensions are those preparations consisting in a dispersed system, comprising two phases, which contain the active principle(s) and additives. One of the phases, the continuous or external phase is generally a liquid or a semisolid and the dispersed or internal phase is comprised of non-soluble solids (active principle(s)) but dispersable in the external phase.

An emulsion is a heterogeneous system comprised of two liquids non-miscible to each other, wherein the dispersed phase is comprised of small droplets distributed in the vehicle in which they are immiscible. The active principle(s) can be in the external or in the internal phase.

Mycoses are skin disorders resulting from "fungus" infestation of which there are three types of mycosis, superficial, intermediate as in *Candida* infections and deep infections. Due to *Candida albicans*, genital mycosis constitute one of the most common causes of vaginal discharge, being the fungus more frequently found as responsible. *Candida albicans* infections do not depend on an external contagion since these microorganism form part of the normal flora of the genital area, being observed that between 20 - 60 % of healthy women are intestinal carriers and about 10% are vaginal carriers. The development of the infection being caused by the occurrence of certain factors that promote the fungal growth.

Clindamycin is methyl-6-amino-7-chloro-6,7,8-trioxy-N-((2S,4R)-1-methyl-4-propylprolyl]-1-thio-L-threo-D-galacto-octopiranoside and it can be represented by formula (I).

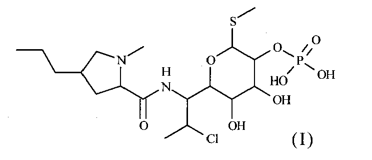

Compound of formula (I) it is known by its antibiotic pharmacological activity, which acts directly by binding to subunit 50S from bacterial ribosome, thereby inhibiting protein synthesis. Its mode of action is primarily as broad spectrum bacteriostatic, more specifically against Gram-positive anaerobic bacteria.

Azoles are heterocyclic, aromatic, 5-membered compounds, wherein nitrogen atoms replace methylene groups, and depending on the number of nitrogen atoms they are classified into azoles, diazoles (Imidazole), triazoles or tetrazoles. These compounds, depending on some changes on the molecule, can be classified as:
1. Imidazoles, which are those that exhibit a difference in the distance between the imidazole and the asymmetric carbon. In addition that the benzene ring attached to the asymmetric carbon exhibits a chlorine atom. (Table II)
2. Triazoles, which are those having a triazole ring and a fluorine atom instead of a chlorine atom on the benzene ring attached to the asymmetric carbon. Also, they exhibit a difference in the distance between the triazole and the asymmetric carbon. (Table III)
3. Oxazoles, which are those that exhibit oxygen.
   from which, imidazoles and triazoles are the most utilized; furthermore, these two sub-families have a great number of compounds, some of which are presented below, without being limiting to the present pharmaceutical formulations. (Table 1)

**TABLE 1**

| IMIDAZOLES | | TRIAZOLES |
|---|---|---|
| Clotrimazole | Miconazole | Itraconazole |
| Tioconazole | Oxiconazole | Fluconazole |
| Econazole | Bifonazole | Terconazole |
| Ketoconazole | Isoconazole | |
| Omoconazole | Butoconazole | |

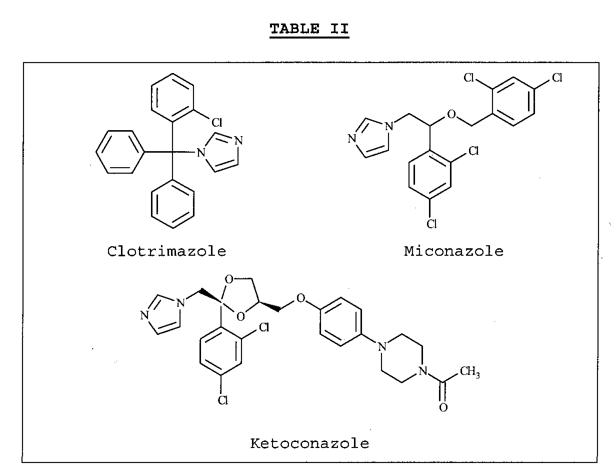

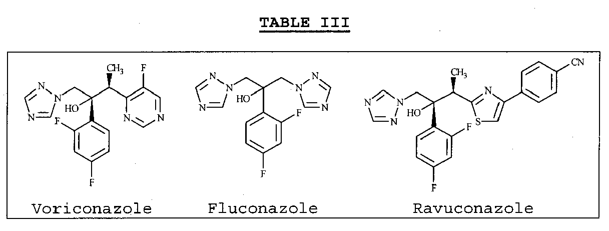

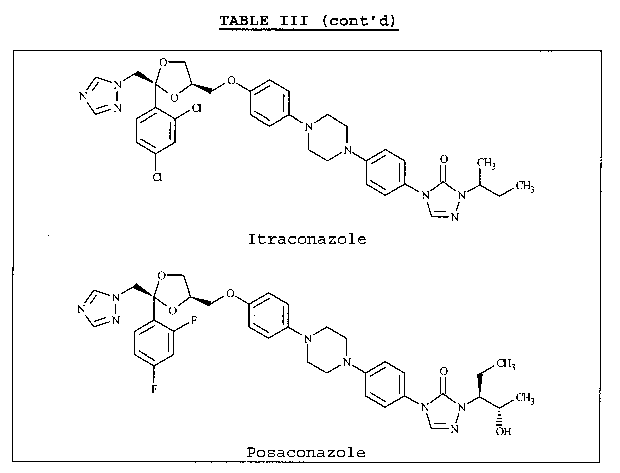

Compounds of tables (II) and (III) are known by their pharmacological activity as antifungal, acting through inhibition of the fungal cytochrome P45014DM activity, also known as lanosterol 14-α-demethylase or CYP51. This enzyme catalyzes the removal of the 14-methyl (c-32) group from lanosterol through three consecutive monooxygenation reactions. Azoles bind to heme group from cytochrome and block lanosterol demethylation into ergosterol, an essential compound of fungal cell wall that regulates the fluidity and permeability of this wall, as well as the activity of enzymes bound to the same. As a consequence of this inhibition the fungal membrane is disrupted and this results in the accumulation of some non-demethylated compounds, thereby causing fungal growth inhibition.

### DESCRIPTION OF THE INVENTION

Therefore, the present invention provides new formulations comprising: (a) Clindamycin, (b) one or more members from the azole family, (c) one or more diluents, (d) one or more disintegrating agents, (e) one or more binding agents, (f) one or more solvents, (g) one or more lubricants, (h) one or more anti-adherents, (i) one or more solubilizing agents, (j) one or more surfactants, (k) one or more emulsifying agents, (1) one or more sweeteners, (m) one or more flavoring agents and/or essences, (n) one or more antioxidants, (o) one or more antimicrobial agents, (p) one or more viscosifying agents, (q) and (r) any other additive that promotes formulation.

The concentration of active principle in the formulation varies from 0.001% to 95.0%, preferably from 0.05 to 40.0% for Clindamycin and from 0.001% to 50.0%, preferably from 0.01 to 50.0% for azoles.

The present invention is characterized in that the drugs combined therein can be present as the anhydrous or hydrated base or as a physiologically acceptable salt.

The pharmaceutical form can contain a series of additives or excipients such as, for example, diluting agents such as lactose, mannitol, dextrose, sucrose, calcium phosphate, microcrystalline cellulose, calcium sulfate, kaolin, compressible sugar, corn starch, among others.

The diluting agent can be present in an amount from 5 to 99%. Disintegrating agents such as corn starch, alginic acid, celluloses and their derivatives, povidone, sodium crosscarmelose, sodium starch glycolate, among others. The disintegrating agent can be present in an amount from 0.0001% to 25.0%.

Binding agents such as polyvinylpyrrolidone, tragacanth, acacia, starch, methyl cellulose, among others. The binding agent can be present in an amount from 0.01% to 10.0%. Polar and non-polar solvents such as: water, ethyl alcohol, acetone, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils such as olive oil, cottonseed oil, corn oil, among others, with ethyl alcohol, 70% sorbitol solution, vegetable oils and polar solvents such as water being the more preferred to be used. The final formulation can contain from 1% to 95% w/v of the dissolvent. Lubricating agents such as stearic acid, stearate and talc, among others.

The lubricant agent can be present in a ratio from 0.0001% to 10.0%.

Anti-adhering agents such as, colloidal silicon dioxide, calcium sulfate, calcium chloride, talc, corn starch, among others. The anti-adherent can be present in a ratio from 0.0001% to 10.0%. Solubilizing agents such as povidone, cyclodextrins, n-methylglucamine, lecithin, monoethanolamine, glycine, benzyl benzoate, poloxamers, polyoxyethylene alquil ethers, among others.

The solubilizing agent can be present in a ratio from 0.0001% to 50.0 %.

One or more surfactants such as sodium laurate, sodium oleate, sodium laurylsulfate, sodium cholate, sodium deoxycholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, among others.

The surfactant can be present in a ratio from 0.0001% to 30%.

Emulsifying agents such as Arabic gum, tragacanth gum, alginates, chondrus, pectin, aluminum silicate, bentonite, aluminum magnesium silicate, sodium dodecylsulfate, benzalconium chloride, polyoxyethylene sorbitan monostereate, polyethylene glycol 400 monostearate, ethylene glycol distearate, polyethylene glycol 400, ethylene glycol distearate, sorbitan tristearate, sorbitan monopalmitate, diethylene glycol monostearate, sodium oleate, potassium oleate, sodium lauryl sulfate, among others. The emulsifying agent can be present from 0.0001% to 10%.

Sweetening agents such as aspartame, acesulfame k, dextrose, fructose, glucose, mannitol, sorbitol, sugar, sucrose, among others. The sweetening agent can be present in a ratio from 0.0001% to 60.0%.

Flavoring agents, such as menthol, vanillin, cinnamon, sorbitol, citric acid, cherry flavor, orange flavor, pineapple flavor, peach flavor, grape flavor, strawberry flavor, among others. The flavoring agent can be present in a ratio from 0.0001% to 5.0%.

Antioxidant agents such as disodium edetate (EDTA), ascorbic acid, butylhydroxytoluene (BHT), tocopherols, sodium bisulfite or sodium metabisulfite, gallic acid, propylgallate, ascorbyl palmitate, among others. The antioxidant agent can be present in a ratio from 0.0001% to 20.0%.

Antimicrobial agents such as sorbic acid; potassium sorbate; potassium benzoate; chlorobutanol; methyl p-hydroxybenzoate; propyl p-hydroxybenzoate; thimerosal, among others. The antimicrobial agent can be present in a ratio from 0.0001% to 5.0% w/v.

Viscosifying agents such as acacia, agar, alginic acid, povidone, hydroxypropylmethyl cellulose, sodium carboxymethyl cellulose, calcium carboxymethylcellulose, pectin, gelatin, guar gum, xanthan gum, carbomer, bentonite, hydroxypropylcellulose, among others, preferably sodium carboxymethyl cellulose. The final formulation can contain from 0.0001% to 10.0% of the viscosifying agent.

The formulation can also contain other components such as a flocculating agent like sodium chloride or potassium chloride; tonicity agents such as sodium chloride, potassium chloride, dextrose, mannitol, among others. A pH buffering system such as phosphates, citrates, carbonates, bicarbonates, acetates, lactates, among others. Alcalinizing or acidifying agents such as sodium hydroxide, sodium bicarbonate, monoethanolamine, triethanolamine, hydrochloric acid, citric acid, acetic acid, among others. Binding agents such as polyvidone, tragacanth, acacia, starch, methyl cellulose, among others. Coatings such as the methacrylates, cellulose derivatives, a blend of polymers and polysaccharides for coating by film formation with aqueous or organic solvents, with coloring agents, flavoring agents, sugars and any other ingredient that is used for film forming, coating and dragee making applications. Soft gelatin or hard gelatin capsules, among others.

The formulations can be contained in containers having suitable capacity varying from 1 ml to 150 ml elaborated from high and/or low density polyethylene, polyethylene terephthalate, polyvinyl chloride, polypropylene, polystyrene, Type I, II, III and IV glass, among others, colored or non colored. The cap may be of the following types: tamperproof, threaded, cap-to-cap, child proof, elaborated from high and/or low density polyethylene, polyethylene terephthalate, polyvinyl chloride, polypropylene, polystyrene, colored or non colored. In a blisterpack made of PVC (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film.

The elaboration of the different pharmaceutical forms is carried out mixing the active principles with the corresponding additives, in the proper concentrations.

Clindamycin is absorbed rapidly and extensively, since almost 90% of the oral dose is absorbed in the gastrointestinal tract, and plasma concentrations from 2 to 3 mg/ml are present one hour after the administration of a 150 mg dose. Its adsorption is not affected by the presence of food in the stomach but it is decreased. Similarly 90% of Clindamycin present in blood is found bound to plasma proteins, and when vaginally administered about 5% is absorbed systemically.

All the azoles are well absorbed after oral administration, which results in a bioavailability close to the maximum (>90%), with the exception of itraconazole, the bioavailability of which fluctuates about 50%. This process is carried out at a fast velocity, reaching the maximum concentration of voriconazole and fluconazole before two hours, whereas with itraconazole it takes up to four hours. The azole-type drugs are widely distributed in the organism, exhibiting distribution volumes of voriconazole between 2 and 4.6 l/kg, itraconazole about 10.7 l/kg and fluconazole between 0.7 and 1 l/kg. These figures greatly outweigh the water content of the body, which indicates greater tissue retention by the drug. With the exception of itraconazole, protein binding is low. Clearance shows the greatest differences between the various azoles; by instance fluconazole is eliminated in a higher proportion in urine, whereas itraconazole and voriconazole are cleared mainly through hepatic metabolism.

For the treatment of vaginal mycoses the use of 80 - 400 mg Clindamycin every 24 hours in adults is recommended. As for the azoles, 100 - 400 mg every 24 hours in adults is recommended. The prescribed treatment for the present invention is 100 mg Clindamycin and 125 mg of one or more members from the azole family per 5 grams of cream every 24 hours in adults.

Therefore the present invention provides a pharmaceutical formulation in solid, semisolid, suspension, solution, emulsion or syrup form which provides from 0.25 mg to 2000.00 mg Clindamycin and from 0.50 mg to 2500.00 mg of one or more members from the azole family in suitable doses.

### EXAMPLES

The following non-limiting examples will assist to illustrate the present invention:

### EXAMPLE 1

| | |
|---|---|
| Azoles | 7.50 % w/w |
| Clindamycin phosphate | 0.13 % w/w |
| Lactose monohydrate | 25.00 % w/w |
| Microcrystalline cellulose PH 101 | 25.00 % w/w |
| Microcrystalline cellulose PH 102 | 35.37 % w/w |
| Sodium starch glycolate | 3.00 % wlw |
| Polyvidone | 3.00 % w/w |
| Magnesium stearate | 1.00 % w/w |

Tablet preparation is performed as follows: Clindamycin phosphate and lactose monohydrate, microcrystalline cellulose PH 101 and sodium starch glicolato are mixed, polyvidone is added and in a separate vessel, microcrystalline cellulose PH 102 and the azole(s) are mixed and the first mixture is gradually added. Finally magnesium stearate is added and mixed. Tableting is carried out.

### EXAMPLE 2

| | |
|---|---|
| Clindamycin phosphate | 6.00 % w/w |
| Azoles | 0.10 % w/w |
| Lactose monohydrate | 20.00 % w/w |
| Microcrystalline cellulose PH 101 | 20.00 % w/w |
| Microcrystalline cellulose PH 102 | 48.10 % w/w |
| Sodium starch glycolate | 2.40 % w/w |
| Polyvidone | 2.40 % w/w |
| Magnesium stearate | 1.00 % w/w |

Powder elaboration is carried out as follows: the azole(s)s are mixed with lactose monohydrate, in a separate vessel, microcrystalline cellulose PH 101 and sodium starch glycolate, polyvidone is added and mixed, to this mixture microcrystalline cellulose PH 102 and Clindamycin phosphate is also added and this mixture is gradually added to the first mixture. Finally, magnesium stearate is added and mixed. Thereafter, the encapsulation is performed.

### EXAMPLE 3

| | |
|---|---|
| Clindamycin base | 6.50 % w/v |
| Azoles | 6.50 % w/v |
| Poloxamer 188 | 1.00 % w/v |
| Sucrose | 30.00 % w/v |
| Sodium Carboxymethyl cellulose | 0.50 % w/v |
| Peach flavoring | 1.50 % w/v |
| Sodium benzoate | 0.25 % w/v |
| Purified water q.s. | 100.00 % w/v |

Suspension is prepared as follows: in a portion of water sucrose and Poloxamer 188 are dissolved; to this mixture Clindamycin base is added and stirred until it is completely incorporated. In another portion of water sodium carboxymethyl cellulose is dispersed and to this peach flavor, the azole(s) and sodium benzoate are added. This mixture is added slowly to the mixture from the first step. Add purified water until the final volume is obtained.

### EXAMPLE 4

| | |
|---|---|
| Clindamycin base | 0.20 % w/w |
| Azoles | 0.05 % wlw |
| Poloxamer 188 | 0.50 % w/w |
| Carbomer | 10.00 % w/w |
| Sodium hydroxyde | 5.00 % w/w |
| 96° Ethyl alcohol | 4.00 % w/w |
| Propylparaben | 2.00 % w/w |
| Methylparaben | 0.01 % w/w |
| Purified water q.s. | 80.00% w/w |

The preparation of an amount of purified water disperse all the Carbomer, once dispersed, separately, dissolve sodium hydroxide and slowly add it to the carbomer with constant stirring. In another portion of purified water add the ethyl alcohol and dissolve the propylparaben, methylparaben, Poloxamer 188, the azole(s) and Clindamycin base. The active(s)-containing solution is added to the matrix and stirred until homogenization.

## Claims

1. Solid pharmaceutical formulations containing Clindamycin comprising (a) one or more members from the azole family, (b) one or more diluents, (c) one or more disintegrating agents, (d) one or more binding agents, (e) one or more solvents, (f) one or more lubricants (g) one or more anti-adherents, (h) one or more solubilizing agents,
(i) one or more surface active agents, (j) one or more emulsifiers, (k) one or more sweeteners, (l) one or more flavoring and/or essences, (m) one or more antioxidants, (n) one or more antimicrobial agents, and (o) one or more viscosifying agents.

2. Solid pharmaceutical formulations containing clindamycin as claimed in precedent claim, wherein drug concentration in the formulation is in a ratio from 0.001% to 95.0%, preferably from 0.05 to 40.0% for Clindamycin and in a ratio from 0.001%4 to 50.0%, preferably from 0.01 to 50.0% for the azoles.

3. Solid pharmaceutical formulations containing clindamycin, as claimed in claim 1 or 2, wherein the drugs combined therein can be present as the anhydrous or hydrated base or as a physiologically acceptable salt.

4. Solid pharmaceutical formulations containing clindamycin, as claimed in any one of claims 1 to 3 wherein said formulations contain a) one or more diluents such as lactose, mannitol, calcium phosphate, microcrystalline cellulose, calcium sulfate, compressible sugar, corn starch, among others; b) disintegrating agents such as corn starch, alginic acid, celluloses and their derivatives, povidone, sodium crosscarmelose, sodium starch glycolate, among others; c) binding agents such as polyvidone, tragacanth, acacia, starch, methyl cellulose, among others; d) polar and non-polar solvents such as: water, ethyl alcohol, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils like olive oil, cottonseed oil, corn oil, among others; e) lubricating agents such as stearic acid, stearate and talc; f) anti-adhering agents such as colloidal silicon dioxide, calcium sulfate, calcium chloride, talc, corn starch, among others.

5. Solid pharmaceutical formulations containing clindamycin, as claimed in any one of claims 1 to 4 wherein the solid pharmaceutical forms can also contain other components such as coatings like the methacrylates, cellulose derivatives, a blend of polymers and polysaccharides for coating by film formation with aqueous or organic solvents, with coloring agents, flavoring agents, sugars and any other ingredient that is used for film forming, coating and dragee making applications. Soft gelatin or hard gelatin capsules, among others.

6. Solid pharmaceutical formulations containing clindamycin, as claimed in any one of claims 1 to 4 wherein the solid pharmaceutical forms can be contained in containers made of a material selected from the group consisting of high and/or low density polyethylene, polyethylene terephthalate, polyvinyl chloride, polypropylene, polystyrene, Type I, II, III and IV glass, colored or non-colored; the cap may be of the type selected from tamperproof cap, threaded, cap-to-cap, child proof, made from a material selected from high and/or low density polyethylene, polyethylene terephthalate, polyvinyl chloride, polypropylene, polystyrene, colored or non-colored; in a container selected from a blisterpack made of PVC (polyvinyl chloride) film with a thickness from 200 µ to 250 µ, non-coated or coated with a PVDC (polyvinylidene chloride) coating having a weight from 25 g/m² to 120 g/m², and bonded with aluminum foil or in cellopolyal film.

7. Solid pharmaceutical formulations containing clindamycin, as claimed in any one of claims 1 to 6 wherein said semisolid, in suspension, in solution, in emulsion or syrup pharmaceutical forms, contain one or more polar and non-polar solvents such as: water, ethyl alcohol, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils like olive oil, cottonseed oil, corn oil, among others, with ethyl alcohol, 70% sorbitol solution, vegetable oils and polar solvents such as water being preferably used; solubilizing agents such as povidone, cyclodextrins, n-methylglucamine, lecithin, monoethanolamine, glycine, benzyl benzoate, poloxamers, polyoxyethylene alkyl ethers, among others; one or more surface active agents such as sodium laurate, sodium oleate, sodium laurylsulfate, sodium cholate, sodium deoxicholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, among others; emulsifying agents such as Arabic gum, tragacanth, alginates, chondrus, pectin, aluminum silicate, bentonite, aluminum magnesium silicate, sodium dodecylsulfate, benzalkonium chloride, polyoxyethylene sorbitan monostearate, polyethylene glycol 400 monostearate, ethylene glycol distearate, sorbitan tristearate, sorbitan monopalmitate, diethyleneglycol monostearate, sodium oleate, potassium oleate, sodium laurylsulfate, among others; sweeteners such as aspartame, acesulfame k, dextrose, fructose, glucose, mannitol, sorbitol, sugar, sucrose, among others; flavoring agents, such as menthol, vanillin, cinnamon, sorbitol, citric acid, cherry flavor, orange flavor, peach flavor, grape flavor, strawberry flavor, among others; antioxidants such as disodium edetate (EDTA), ascorbic acid, Butylhydroxytoluene (BHT), tocopherols, sodium bisulfite or sodium metabisulfite, gallic acid, propylgallate, ascorbyl palmitate among others; antimicrobial agents such as sorbic acid, potassium benzoate, chlorobutanol, methyl p-hydroxybenzoate, propyl p-hydroxybenzoate, thimerosal among others; viscosifying agents like acacia, agar, alginic acid, povidone, hydroxypropylmethyl cellulose, sodium carboxymethyl cellulose, calcium carboxymethyl cellulose, pectin, gelatin, guar gum, xanthan gum, carbomer, bentonite, hydroxypropyl cellulose, among others.

8. Solid pharmaceutical formulations containing clindamycin, as claimed in any one of claims 1 to 7 wherein said semisolid, in suspension, in solution, in emulsion or syrup pharmaceutical forms can also contain other components such as a flocculating agent like sodium chloride or potassium chloride; tonicity agents such as sodium chloride, potassium chloride, dextrose, mannitol, among others; a pH buffering system such as phosphates, citrates, carbonates, bicarbonates, acetates, lactates, among others; alcalinizing or acidifying agents such as sodium hydroxide, sodium bicarbonate, monoethanolamine, triethanolamine, hydrochloric acid, citric acid, acetic acid, among others; binding agents such as polyvidone, tragacanth, acacia, starch, methyl cellulose, among others.

9. Solid pharmaceutical formulations containing clindamycin, as claimed in claim 8 wherein said formulations are contained in bottles of suitable capacity varying from 1 ml to 150 ml, elaborated from high and/or low density polyethylene, polyethylene terephthalate, polyvinyl chloride, polypropylene, polystyrene, Type I, II, III and IV glass, among others, colored or non-colored, the cap can be tamperproof, threaded, cap-to-cap, child proof, elaborated from high and/or low density polyethylene, polyethylene terephthalate, polyvinyl chloride, polypropylene, polystyrene, colored or non-colored; dosing auxiliaries, such as dosing spoons, metering cups, pipettes, small syringes, inserts elaborated from materials such as, inter alia, high and/or low density polyethylene, polyethylene terephthalate, polyvinyl chloride, polypropylene, polystyrene, colored or non-colored, are also included.

10. Solid pharmaceutical formulations containing clindamycin, as claimed in claim 8 wherein they provide from 0.25 g to 2000.00 mg of Clindamycin and from 0.50 mg to 2500.00 mg of one or more members from the azoles family in suitable doses.
